# EUROPEAN PATENT APPLICATION

(11) **EP 4 796 525 A1**
(43) Date of publication of application: **26.08.2026**
(21) Application number: 24879484.4
(22) Date of filing: 18.09.2024
(51) Int. Cl.: C07C 67/26, C07C 69/54

(54) **METHOD FOR PRODUCING MONOMER PRECURSOR COMPOSITION**

(30) Priority: 18.10.2023 JP 2023179234
(71) Applicant: Toray Industries, Inc., Tokyo 103-8666 (JP)
(72) Inventor: TAKEDA, Keishi, Otsu-shi, Shiga 520-8558 (JP)
(74) Representative: Hoefer & Partner Patentanwälte mbB
(86) International application number: PCT/JP2024/033202
(87) International publication number: WO 2025/084057

(57) **Abstract**

The present invention pertains to a method for producing a precursor to be used in a monomer which can be easily prepared, which has high versatility in designing, and which is for use in medical devices having high transparency, especially contact lenses. The present invention provides a method for producing a monomer precursor composition, the method comprising a mixing step for mixing: a compound A having a specific structure (a); an unsaturated carboxylic acid B having a specific structure (b); an unsaturated carboxylic acid metal salt C; and an unsaturated carboxylic acid D having a substituent group selected from the group consisting of halogenated alkyl groups, isocyanate groups, carboxyl groups, epoxy groups, acid anhydride groups, and carboxylic acid halide groups.

## Description

### TECHNICAL FIELD

The present invention relates to a production method for a monomer precursor composition.

### BACKGROUND ART

Conventionally, there are known polymers that can be produced by polymerizing (meth)acrylic monomers and serve as medical materials. These polymers are sufficiently high in transparency and in hardness and accordingly, they have been used particularly for ophthalmic applications and dental applications.

For example, compounds as represented by the formula (c) or (c') given below have been known as such (meth)acrylic monomers (Patent document 1). These compounds possess two types of polymerizable groups, and accordingly, they can be used as crosslinking agents. In particular, when they are used as crosslinking agents for producing hydrogel materials, both types of them are characterized by being high in compatibility with hydrophilic monomers such as hydroxymethyl methacrylate (HEMA) because they have hydroxyl groups.

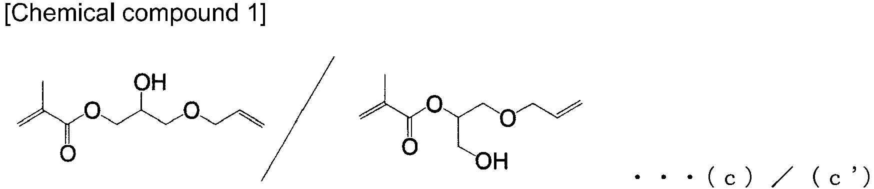

On the other hand, in the case of compounds that are represented by the formula (c) or (c') given above, it is unavoidable that compounds as represented by the formula (d) given below become mixed therein as by-products generated in the course of the process of their production. These by-products that are represented by the formula (d) given below possess three types of polymerizable groups within their molecules, and therefore they also act as crosslinking agents. However, if the intrusion of these by-products, which act as crosslinking agents having physical properties different from those of the main compounds, occurs to an inappropriately high degree, it becomes difficult to control the polymerization reaction in the originally intended manner, resulting in extremely unstable quality control of the hydrogel material etc. to be produced.

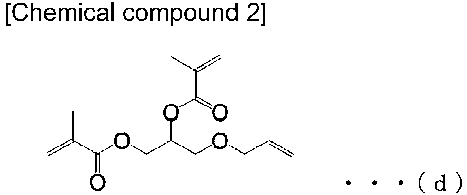

For example, in order to reduce the generation of such by-products, it may be effective to purify the crosslinking agent used as a main component, but such a method may cause a decrease in the yield of the main component. In addition, as a way of increasing the amount of a by-product that can get mixed in, it is conceivable that such a by-product is prepared separately and added afterward, but an additional step will be necessary for preparing that by-product.

As exemplified above, when producing a (meth)acrylic monomer that can be used as a crosslinking agent, the only available method in the current situation is to control the generation of by-products as effectively as possible.

### PRIOR ART DOCUMENTS

### PATENT DOCUMENTS

Patent document 1: Japanese Patent No. 4617887

### SUMMARY OF INVENTION

### PROBLEMS TO BE SOLVED BY THE INVENTION

In view of the foregoing, the main object of the present invention is to provide a production method for a monomer precursor composition which, in the production of a precursor of an acrylic monomer suitable as a component for medical materials, makes it possible to control the amount of by-products to a desired level by means of a simple procedure.

### MEANS OF SOLVING THE PROBLEMS

In order to achieve the above object, the present invention provides a production method for a monomer precursor composition that includes a step designed to mix a compound A as represented by the general formula (a) given below, an unsaturated carboxylic acid B as represented by the general formula (b) given below, an unsaturated carboxylic acid metal salt C, and an unsaturated carboxylic acid D having a substituent selected from the group consisting of a halogenated alkyl group, an isocyanate group, a carboxyl group, an epoxy group, an acid anhydride group, and a carboxylic acid halide group.

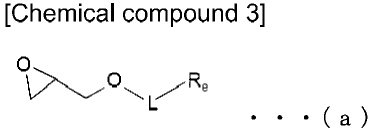

[In the general formula (a), L represents a linear or branched alkylene group having 1 to 10 carbon atoms, and Rₑ represents a linear or branched hydrocarbon group having 2 to 10 carbon atoms and containing an unsaturated bond.]

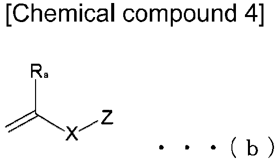

[In the general formula (b), Rₐ represents a hydrogen atom or a methyl group; X represents a single bond or a linear or branched alkylene group having 1 to 10 carbon atoms; and Z represents a carboxyl group.]

### ADVANTAGEOUS EFFECTS OF THE INVENTION

According to the present invention, it is possible to control the generation of by-products by means of an extremely simple procedure involving only a one-step reaction, and thereby it is possible to produce a monomer precursor composition having a specific structure and containing an appropriate amount of by-products.

### DESCRIPTION OF PREFERRED EMBODIMENTS

The production method for a monomer precursor composition according to the present invention requires the inclusion of a mixing step designed to mix a compound A as represented by the general formula (a) given below, an unsaturated carboxylic acid B as represented by the general formula (b) given below, an unsaturated carboxylic acid metal salt C, and an unsaturated carboxylic acid D having a substituent selected from the group consisting of a halogenated alkyl group, an isocyanate group, a carboxyl group, an epoxy group, an acid anhydride group, and a carboxylic acid halide group.

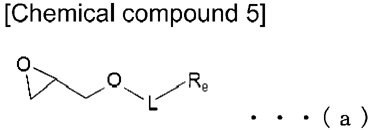

In the general formula (a), L represents a linear or branched alkylene group having 1 to 10 carbon atoms, and Rₑ represents a linear or branched hydrocarbon group having 2 to 10 carbon atoms and containing an unsaturated bond.

From the viewpoint of imparting high flexibility to the final polymer product intended for use as medical material, it is preferable that L have 1 to 8 carbon atoms, more preferably 1 to 4 carbon atoms. From a similar viewpoint, it is preferable that Rₑ have 2 to 8 carbon atoms, more preferably 2 to 4 carbon atoms. Here, the term 'unsaturated bond' refers to a carbon-carbon double bond that can cause radical polymerization.

For the present invention, the compound A that is represented by the general formula (a) is preferably an aliphatic glycidyl ether monomer, and it is particularly preferably an allyl glycidyl ether from the viewpoint of ease of availability.

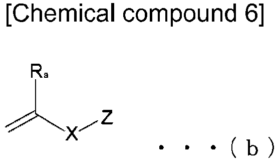

In the general formula (b), Rₐ represents a hydrogen atom or a methyl group; X represents a single bond or a linear or branched alkylene group having 1 to 10 carbon atoms; and Z represents a carboxyl group.

For the present invention, examples of the unsaturated carboxylic acid B that is represented by the general formula (b) include vinyl oxyacetic acid, allyloxyacetic acid, (meth)acrylic acid, crotonic acid, 2-(meth)acryloylpropanoic acid, 3-(meth)acryloylbutanoic acid, and 4-vinylbenzoic acid, of which (meth)acrylic acid is preferred from the viewpoint of ease of availability.

For the mixing step included in the production method for a monomer precursor composition according to the present invention, the amount of the unsaturated carboxylic acid B used therein is preferably 1.0 to 50 equivalents, more preferably 1.5 to 40 equivalents, and still more preferably 2.0 to 30 equivalents, relative to the compound A that is represented by the general formula (a) of the present invention, from the viewpoint of increasing the reaction rate of the compound A that is represented by the general formula (a) of the present invention.

For the present invention, a compound A as represented by the general formula (a) is difficult to remove by using any of various available purification methods although its content in the monomer precursor composition is preferably as small as possible because it may leach out in due course if it remains in the final polymer product, that is, in the intended medical material. The content of the compound A represented by the general formula (a) of the present invention present in the resulting monomer precursor composition is preferably 1,500 ppm or less. For use as a medical material, it is more preferably 1,000 ppm or less and still more preferably 500 ppm or less.

The term "unsaturated carboxylic acid metal salt C" used for the present invention refers to a metal salt of an unsaturated carboxylic acid that has a carboxyl group. Examples of such a metal salt include salts of alkali metals such as sodium, potassium, and lithium, and salts of alkaline earth metals such as calcium. In particular, from the viewpoint of reactivity and ease of availability, salts of alkali metals, that is, alkali metal salts, are preferable, and salts of sodium ion and lithium ion are more preferable. Furthermore, from the viewpoint of suppressing the generation of impurities, it is preferable that the unsaturated carboxylic acid metal salt C be a metal salt of an unsaturated carboxylic acid B as represented by the general formula (b) of the present invention.

In the mixing step included in the production method for a monomer precursor composition according to the present invention, it is preferable that the unsaturated carboxylic acid metal salt C added above act as a catalyst.

For the mixing step, the amount of the unsaturated carboxylic acid metal salt C added therein is preferably 0.001 to 5.000 equivalent, more preferably 0.005 to 3.000 equivalents, and still more preferably 0.010 to 1.000 equivalents, relative to the compound A that is represented by the general formula (a) of the present invention, from the viewpoint of ensuring an appropriate reaction rate and preventing precipitation thereof.

The unsaturated carboxylic acid D to use for the present invention has a substituent selected from the group consisting of a halogenated alkyl group, an isocyanate group, a carboxyl group, an epoxy group, an acid anhydride group, and a carboxylic acid halide group.

Examples of preferred functional groups present in the unsaturated carboxylic acid D include isocyanate groups, epoxy groups, acid anhydride groups, and carboxylic acid halide groups from the viewpoint of reaction convenience in terms of suppression of formation of by-products other than desired ones. Among these, the presence of an isocyanate group and an acid anhydride group is more preferable, and the use of an acid anhydride group is still more preferable.

Examples of the unsaturated carboxylic acid D having an isocyanate group include 2-(meth)acryloyloxyethyl isocyanate, 3-methacryloyloxy-n-propyl isocyanate, 2-methacryloyloxyisopropyl isocyanate, 4-methacryloyloxy-n-butyl isocyanate, 2-methacryloyloxy-tert-butyl isocyanate, 2-methacryloyloxybutyl-4-isocyanate, 2-methacryloyloxybutyl-3-isocyanate, 2-methacryloyloxybutyl-2-isocyanate, 2-methacryloyloxybutyl-1-isocyanate, 5-methacryloyloxy-n-pentyl isocyanate, 6-methacryloyloxy-n-hexyl isocyanate, 7-methacryloyloxy-n-heptyl isocyanate, 2-(isocyanatoethyloxy)ethyl methacrylate, 3-methacryloyloxyphenyl isocyanate, and 4-methacryloyloxyphenyl isocyanate, in which the methacryl groups may be replaced with acryl groups. Among these, 2-(meth)acryloyloxyethyl isocyanate is preferable from the viewpoint of ease of availability.

Preferred examples of the unsaturated carboxylic acid D having an acid anhydride group, i.e., an unsaturated carboxylic acid D that is in the form of an acid anhydride, include acetic anhydride, (meth)acrylic anhydride, succinic anhydride, phthalic anhydride, maleic anhydride, benzoic anhydride, itaconic anhydride, n-decylsuccinic anhydride, n-dodecylsuccinic anhydride, n-tetradecylsuccinic anhydride, and n-hexadecylsuccinic anhydride, of which (meth)acrylic anhydride is preferable from the viewpoint of ease of availability.

For the mixing step included in the production method for a monomer precursor composition according to the present invention, the amount of the unsaturated carboxylic acid D used therein is preferably 0.001 to 30 mass%, more preferably 0.005 to 25 mass%, and still more preferably 0.010 to 20 mass%, relative to the compound A that is represented by the general formula (a) of the present invention, from the viewpoint of ensuring an appropriate balance between the reaction rate and the reactivity in the mixing step.

The monomer precursor composition to be produced according to the present invention is a composition that contains, as the main component, a monomer precursor as represented by the general formula (e) given below that is synthesized by the production method according to the present invention, and also contains, as a by-product, a compound as represented by the general formula (e') given below.

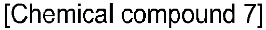

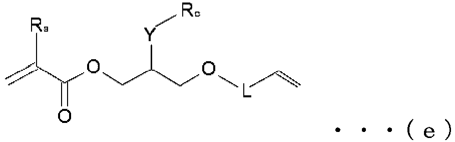

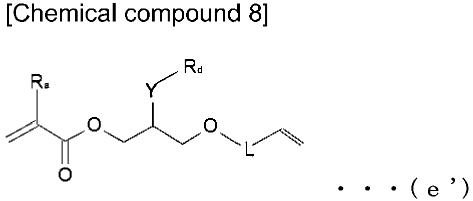

In the general formulae (e) and (e'), Rₐ represents a hydrogen atom or a methyl group, and L represents a linear or branched alkylene group having 1 to 10 carbon atoms. Here, from the viewpoint of imparting high flexibility to the final polymer product, the number of carbon atoms in L is preferably 1 to 8 and more preferably 1 to 4.

R_{c} represents a hydrogen atom or a linear or branched hydrocarbon group having 1 to 10 carbon atoms, but from the viewpoint of avoiding steric hindrance, it is preferable that R_{c} be a hydrogen atom or a hydrocarbon group having 2 to 4 carbon atoms.

R_{d} represents a linear or branched hydrocarbon group having an unsaturated bond and 1 to 10 carbon atoms, but from the viewpoint of avoiding steric hindrance, it is preferable that R_{d} be a hydrocarbon group having 1 to 10 carbon atoms, more preferably a hydrocarbon group having 2 to 8 carbon atoms, and still more preferably a hydrocarbon group having 2 to 4 carbon atoms.

Furthermore, the amount of the compound represented by the general formula (e') that is present in the monomer precursor composition may be adjusted as appropriate depending on the properties required in the final polymer product, but from the viewpoint of ensuring ease of molding, it is preferably 0.001 to 40 mol%, more preferably 0.5 to 30 mol%, and still more preferably 2.0 to 20 mol%, relative to the amount of the monomer precursor represented by the general formula (e).

Those compounds represented by the general formula (e) or (e') have structures formed by first adding an unsaturated carboxylic acid B as represented by the general formula (b) of the present invention to a compound A as represented by the general formula (a) of the present invention, followed by a reaction thereof mainly with the functional group present in the unsaturated carboxylic acid D used for the present invention.

It is preferable for Y to have a structure that is represented by any of the general formulae (f) given below.

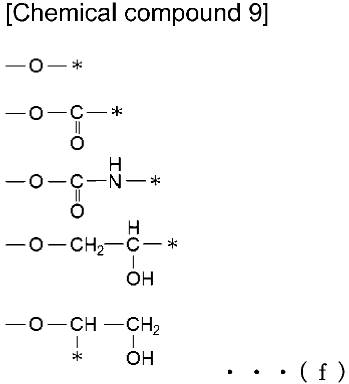

In the structures represented by the general formulae (f), "*" represents a covalent bond with R_{c} or R_{d}.

From the viewpoint of reactivity with the functional group present in the unsaturated carboxylic acid D, it is more preferable that Y have a structure as represented by any of the formulae (f2) given below.

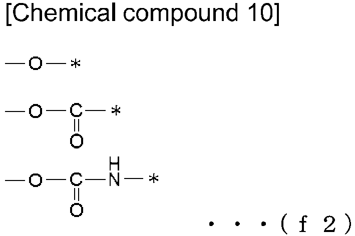

In the structures represented by the general formulae (f2), "*" represents a covalent bond with R_{c} or R_{d}.

In the mixing step included in the production method for a monomer precursor composition according to the present invention, a polymerization inhibitor may be added for the purpose of preventing the polymerization of the resulting monomer precursor or the like. Examples of good polymerization inhibitors include hydroquinone based compounds such as hydroquinone, methylhydroquinone, t-butylhydroquinone, 2,6-di-t-butyl-4-methylphenol, and hydroquinone monomethyl ether; nitroso compounds such as p-nitrosophenol, nitrosobenzene, N-nitrosodiphenylamine, isononyl nitrite, N-nitrosocyclohexylhydroxylamine, N-nitrosophenylhydroxylamine, N,N'-dinitrosophenylenediamine, and salts thereof; nitrone compounds such as α-phenyl-N-t-butylnitrone and α-naphthyl-N-t-butylnitrone; and nitroxide compounds such as 2,2,6,6-tetramethyl-1-piperidinyloxide (TEMPO) and 4-hydroxy-2,2,6,6-tetramethyl-1-piperidinyloxide.

From the viewpoint of ensuring a sufficient polymerization inhibiting effect without inhibiting the polymerization for forming the finally intended polymer, the amount of the polymerization inhibitor to be added is preferably 0.0005 to 5 equivalents, more preferably 0.0010 to 3 equivalents, and still more preferably 0.0050 to 1 equivalent, relative to the amount of the compound A that is represented by the general formula (a) of the present invention.

For the mixing step included in the production method for a monomer precursor composition according to the present invention, it is preferable, from the viewpoint of ensuring an appropriate balance between the reaction time and the reactivity, that the temperature used therein be 50°C to 180°C, more preferably 60°C to 170°C, and still more preferably 70°C to 160°C.

The production method for a monomer precursor composition according to the present invention may further include a washing step designed for washing the monomer precursor composition resulting from the mixing step. This washing step serves for the removal of unreacted raw materials, impurities formed during the reaction, and the like. The procedure to perform the washing step may include, for example, adding an organic solvent to the monomer precursor composition formed and subsequently adding an aqueous solution of an alkali or an electrolyte, or pure water, followed by mixing, shaking, and leaving stationary the resulting mixture to cause liquid separation. Examples of good organic solvents to use here include aromatic organic solvents such as benzene, toluene, and xylene; halogenated organic solvents such as dichloromethane, chloroform, and dichloroethane; ketone based organic solvents such as methyl isobutyl ketone, diethyl ketone, and cyclohexanone; and ester based solvents such as n-butyl acetate and isobutyl acetate.

A monomer precursor composition that can be produced by the production method according to the present invention may be used in the as-produced form or may be used after adding a compound having an alkyl group or aryl group possessing a siloxanyl group, hydroxyl group, carboxyl group, amino group, or the like to the active terminal group present in the monomer precursor.

### EXAMPLES

The present invention will be illustrated below in greater detail with reference to examples, but the invention should not be construed as being limited thereto.

### <Measuring method>

Gas chromatographic (GC) measurement was performed using a GC-2014 unit (FID detector) manufactured by Shimadzu Corporation as the main device in combination with a ULTRA-2 capillary column (0.32 mm × 30 m × 0.52 µm) manufactured by J&W. The carrier gas used was a mixed gas of helium and nitrogen (110 kPa) and measurement was performed under conditions including an injection port temperature of 300°C, a detector temperature of 320°C, and the use of a temperature elevation program operated in the order of 50°C (1 min), 10°C/min, and 300°C (14 min). A sample was prepared by dissolving 100 µL of a test material in 1 mL of acetonitrile, and 1 µL of the sample was injected.

### <Reaction rate>

A gas chromatograph (GC) was used for the measurement. For each reaction liquid, the reaction rate was calculated from the ratio between peak areas of allyl glycidyl ether measured in the initial stage of the reaction and after stirring for 6 hours.

### <GC purity, (e')/(e)>

Measurement was carried out by using a gas chromatograph (GC). The reaction liquid obtained finally was analyzed, and the peak percentage of the main component, that is, the monomer precursor represented by general formula (e), was determined by the area percentage method and adopted to represent the GC purity. Furthermore, the ratio of (e')/(e) was calculated from the peak percentages of the components represented by the general formulae (e) and (e') by the area percentage method to represent the amount of by-products generated.

### <Yield>

The yield was calculated by dividing the molar mass of the monomer precursor composition finally obtained by the molar mass of the allyl glycidyl ether component used as raw material.

### [Example 1]

To a 100 mL eggplant flask, 5 g of allyl glycidyl ether (43.8 mmol), 8.0 g of methacrylic acid (92.9 mmol), 0.80 g of sodium methacrylate (7.4 mmol), 0.09 g of methacrylic anhydride (0.6 mmol), and 0.07 g of 4-methoxyphenol (0.6 mmol) were fed, stirred for 6 hours in a water bath maintained at 85°C, and then cooled to room temperature, followed by adding 14.8 g of toluene (160.6 mmol). The mixture was washed four times with 25 g of a 2 mass% aqueous solution of sodium hydroxide and twice with 25 g of 15 mass% brine, and then a polymerization inhibitor was added, followed by removal of the solvent using an evaporator. The purity of the resulting liquid was determined based on GC analysis, which gave the results shown in Table 1.

### [Example 2]

To a 100 mL eggplant flask, 5 g of allyl glycidyl ether (43.8 mmol), 8.0 g of methacrylic acid (92.9 mmol), 0.80 g of sodium methacrylate (7.4 mmol), 0.26 g of methacrylic anhydride (1.6 mmol), and 0.07 g of 4-methoxyphenol (0.6 mmol) were fed, stirred for 6 hours in a water bath maintained at 85°C, and then cooled to room temperature, followed by adding 14.8 g of toluene (160.6 mmol). The mixture was washed four times with 25 g of a 2 mass% aqueous solution of sodium hydroxide and twice with 25 g of 15 mass% brine, and then a polymerization inhibitor was added, followed by removal of the solvent using an evaporator. The purity of the resulting liquid was determined based on GC analysis, which gave the results shown in Table 1.

### [Example 3]

To a 100 mL eggplant flask, 5 g of allyl glycidyl ether (43.8 mmol), 8.0 g of methacrylic acid (92.9 mmol), 0.80 g of sodium methacrylate (7.4 mmol), 0.40 g of methacrylic anhydride (2.6 mmol), and 0.07 g of 4-methoxyphenol (0.6 mmol) were fed, stirred for 6 hours in a water bath maintained at 85°C, and then cooled to room temperature, followed by adding 14.8 g of toluene (160.6 mmol). The mixture was washed four times with 25 g of a 2 mass% aqueous solution of sodium hydroxide and twice with 25 g of 15 mass% brine, and then a polymerization inhibitor was added, followed by removal of the solvent using an evaporator. The purity of the resulting liquid was determined based on GC analysis, which gave the results shown in Table 1.

### [Example 4]

Except that 2-isocyanatoethyl acrylate was used in place of methacrylic anhydride, the same testing procedure as in Example 1 was carried out. The purity of the resulting liquid was determined based on GC analysis, which gave the results shown in Table 1.

### [Example 5]

Except that methacryloyl chloride was used in place of methacrylic anhydride, the same testing procedure as in Example 1 was carried out. The purity of the resulting liquid was determined based on GC analysis, which gave the results shown in Table 1.

### [Comparative example 1]

To a 100 mL eggplant flask, 5 g of allyl glycidyl ether (43.8 mmol), 15.0 g of methacrylic acid (174.2 mmol), 1.42 g of sodium methacrylate (13.2 mmol), and 0.07 g of 4-methoxyphenol (0.6 mmol) were fed, stirred for 6 hours in a water bath maintained at 85°C, and then cooled to room temperature, followed by adding 18.6 g of toluene (201.8 mmol). The mixture was washed with 42.8 g of a 2 mass% aqueous solution of sodium hydroxide and with 42.8 g of 15 mass% brine, and then a polymerization inhibitor was added, followed by removal of the solvent using an evaporator. The purity of the resulting liquid was determined based on GC analysis, which gave the results shown in Table 1.

### [Comparative example 2]

Except that 6.8 g of methacrylic acid (79.0 mmol) and 0.45 g of sodium methacrylate (4.2 mmol) were fed as raw material components and that the addition of methacrylic anhydride was omitted, the same testing procedure as in Example 1 was carried out.

### [Table 1]

**[Table 1]**

| | Reaction rate [%] | GC purity [%] | (e')/(e) [%] | Yield [%] |
|---|---|---|---|---|
| Example 1 | 96.4 | 94.0 | 2.3 | 72 |
| Example 2 | 98.5 | 90.6 | 5.5 | 73 |
| Example 3 | 96.6 | 86.8 | 9.1 | 73 |
| Example 4 | 97.5 | 93.4 | 2.7 | 74 |
| Example 5 | 96.2 | 82.3 | 12.5 | 76 |
| Comparative example 1 | 98.9 | 95.4 | 1.6 | 68 |
| Comparative example 2 | 87.0 | 93.8 | 1.5 | 61 |

## Claims

1. A production method for a monomer precursor composition comprising a step designed to mix a compound A as represented by the general formula (a) given below, an unsaturated carboxylic acid B as represented by the general formula (b) given below, an unsaturated carboxylic acid metal salt C, and an unsaturated carboxylic acid D having a substituent selected from the group consisting of a halogenated alkyl group, an isocyanate group, a carboxyl group, an epoxy group, an acid anhydride group, and a carboxylic acid halide group: 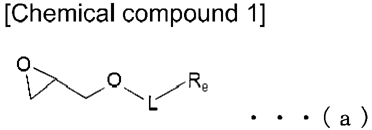
wherein, in the general formula (a), L represents a linear or branched alkylene group having 1 to 10 carbon atoms and Rₑ represents a linear or branched hydrocarbon group having 2 to 10 carbon atoms and containing an unsaturated bond, and 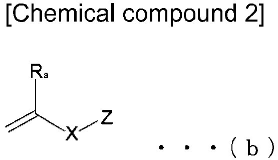
wherein, in the general formula (b), Rₐ represents a hydrogen atom or a methyl group; X represents a single bond or a linear or branched alkylene group having 1 to 10 carbon atoms; and Z represents a carboxyl group.

2. A production method for a monomer precursor composition as set forth in claim 1, wherein the unsaturated carboxylic acid metal salt C is a metal salt of the unsaturated carboxylic acid B.

3. A production method for a monomer precursor composition as set forth in either claim 1 or 2, wherein the unsaturated carboxylic acid metal salt C is a salt of an alkali metal.

4. A production method for a monomer precursor composition as set forth in either claim 1 or 2, wherein the unsaturated carboxylic acid D is an acid anhydride.
